# EUROPEAN PATENT SPECIFICATION

(11) **EP 1 799 754 B1**
(45) Date of publication and mention of the grant of the patent: **19.03.2008**
(21) Application number: 05782673.7
(22) Date of filing: 02.08.2005
(51) Int. Cl.: C08J 7/00, A61L 15/22, A61K 9/70

(54) **POLYMERIC FILM**
POLYMERFOLIE
FILM POLYMERE

(30) Priority: 25.08.2004 US 604265 P
(43) Date of publication of application: 27.06.2007
(73) Proprietor: UNION CARBIDE CHEMICALS & PLASTICS TECHNOLOGY CORPORATION, Danbury, Connecticut 06817-0001 (US)
(72) Inventor: HE, Vicky, Yafen, Brooklyn, NY 11204 (US); SCHMITT, Robert, Louis, Annadale, NY 08801 (US); NGUYEN, Phuc, Garden Grove, CA 92844 (US)
(74) Representative: Schaad, Balass, Menzl & Partner AG
(86) International application number: PCT/US2005/027521
(87) International publication number: WO 2006/026040

(56) References cited:
- EP-A- 0 251 774
- WO-A-03/086493
- US-A- 5 143 071

## Description

The present invention relates to a polymeric film comprising an alkylene oxide homo- or copolymer.

### Background of the Invention

Alkylene oxide polymers are useful in a large variety of applications. U.S. Patent No. 3,264,202 discloses polyalkylene oxides which are cross-linked by ionizing radiation treatment. U.S. Patent No. 3,419,006 discloses transparent wound dressings which comprise a layer of a hydrophilic polymer gel, such as an ethylene oxide polymer cross-linked by irradiation. The article "Ultraviolet-Induced Crosslinking of Solid Poly(ethylene oxide)" by M. Doytcheva et al. in J. Appl. Polym. Sci 64: 2299-2307, 1997 describes ultraviolet light radiation with and without benzophenone as a photoinitiator. The article discloses that cross-linked poly(ethylene oxides) are useful as wound dressings, controlled-release drug systems, phase transfer catalysts, semi-permeable membranes or solid electrolytes for batteries.

Cross-linked poly(ethylene oxides) generally are water-insoluble and do not exhibit significant adhesive properties. However, for certain applications like wound dressings or controlled-release drug systems it would be desirable to provide films which comprise cross-linked C₂₋₄ alkylene oxide homo- or copolymers and which have adhesive properties.

### Summary of the Invention

One aspect of the present invention is a polymeric film comprising an alkylene oxide homo- or copolymer, wherein the film comprises i) a coherent pattern of cross-linked alkylene oxide homo- or copolymer and ii) areas of non-crosslinked alkylene oxide homo- or copolymer at one or both sides of the film.

Another aspect of the present invention is a composite article which comprises an above-mentioned film.

Yet another aspect of the present invention is a process for preparing the above-mentioned film comprising the steps of a) preparing a film comprising an alkylene oxide homo- or copolymer, b) shielding the film at one or both sides of the film at selected areas from irradiation such that a coherent pattern of non-shielded film material remains and c) subjecting the film to irradiation at one or both sides of the film.

Yet another aspect of the present invention is the use of the above-mentioned film as an adhesive film or for preparing an adhesive film.

Yet another aspect of the present invention is the use of the above-mentioned film for preparing a wound dressing film or a drug delivery film.

### Short Description of the Drawings

Figures 1 and 2 represent schematic views on two preferred embodiments of the film of the present invention.
Figure 3 illustrates the swelling of the film of the present invention in the presence of water.

### Detailed Description of the Invention

The film of the present invention comprises a coherent pattern of cross-linked alkylene oxide homo- or copolymer and areas of non-crosslinked alkylene oxide homo- or copolymer.

The cross-linked alkylene oxide homo- or copolymer comprises intra- and intermolecular carbon to carbon cross-linking bonds such that the cross-linked polymer is water-insoluble and stable in the presence of oxygen. The term "water-insoluble" as used herein means that less than 5 grams, preferably less than 2 grams, more preferably less than 1 gram of the cross-linked polymer is soluble in 100 grams of distilled water at 25 °C and 1 atmosphere. The cross-linked polymer forms a matrix, also called continuous phase, that is typically swellable but insoluble in water and that has sufficient film strength even if the film is contacted with water.

The areas of non-crosslinked alkylene oxide homo- or copolymer have adhesive properties when they are contacted with water. The size, number and pattern of adhesive areas of non-crosslinked polymer in the matrix of cross-linked polymer can be varied to a large degree and adjusted to the desired end-use. By providing a film which comprises a coherent pattern of cross-linked alkylene oxide homo- or copolymer and areas of non-crosslinked alkylene oxide homo- or copolymer, an optimal combination of film integrity in me presence or water and adhesiveness is achieved. Moreover, the process of the present invention described below allows the production of a film wherein the integrity of the film in the presence of water and the adhesiveness of the film can be controlled and adjusted to the desired end-use. The non-crosslinked alkylene homo- or copolymer generally has a water-solubility of at least 5 grams, preferably at least 10 grams in 100 grams of distilled water at 25 °C and 1 atmosphere. The non-cross-linked alkylene homo- or copolymer preferably has a weight average molecular weight of from 100,000 to 8,000,000, more preferably from 600,000 to 5,000,000, most preferably from 900,000 to 4,000,000 determined by gel permeation chromatography.

Alkylene oxide homo- or copolymers which are useful in the film of the present invention are preferably C₂₋₄ alkylene oxide homo- or copolymers, such as homo- or copolymers of ethylene oxide, propylene oxide, 1,2-butene epoxide or isobutylene oxide. The film of the present invention most preferably comprises an ethylene oxide homo- or copolymer. Other useful polymers are homo- and copolymers of cycloaliphatic epoxides, such as 1,2-cyclohexene epoxide, vinyl cyclohexene oxides, such as 4-vinyl-1-cyclohexene 1,2-epoxide, epoxycyclohexene or 4-vinyl-1-cyclohexene diepoxide; dipentene epoxide, unsaturated glycidyl ethers, such as vinylglycidyl ether or allyl glycidyl ether; alkyl glycidyl ethers, such as methyl glycidyl ether, ethyl glycidyl ether, isopropyl glycidyl ether, isobutyl glycidyl ether, tert-butyl glycidyl ether, n-hexyl glycidyl ether or n-octyl glycidyl ether; 1,3-butadiene diepoxide, styrene oxide, phenyl glycidyl ether or alkyl phenyl glycidyl ethers.

The alkylene oxide copolymers may be random copolymers produced by the polymerization of mixtures of at least two alkylene oxides. Other useful alkylene oxide copolymers are block copolymers produced by the sequential addition of more than one alkylene oxide, in which nearly total consumption of each alkylene oxide takes place prior to the addition of subsequent monomer(s). Alternatively, the alkylene oxide copolymer may comprise in copolymerized form an alkylene oxide and another copolymerizable monomer, such as methyl acrylate, ethyl acrylate, a caprolactone, ethylene carbonate, trimethylene carbonate, 1,3-dioxolane, carbon dioxide, carbonyl sulfide, tetrahydrofuran, methyl isocyanate, or methyl isocyanide. Preferred alkylene oxide copolymers are copolymers of ethylene oxide with epichlorohydrin or copolymers of ethylene oxide with cyclohexene oxide. Alkylene oxide copolymers generally comprise at least 50 mole percent, preferably at least 70 mole percent, more preferably at least 85 mole percent alkylene oxide units. The most preferred alkylene oxide polymers are ethylene oxide copolymers or, more preferably, ethylene oxide homopolymers.

The film of the present invention preferably comprises at least 40 weight percent, more preferably at least 60 percent, most preferably at least 90 weight percent of an alkylene oxide homo- or copolymer, based on the total weight of the film.

The film may comprise one or more other polymers, preferably water-soluble polymers, for example one or more gums, such as gum arabic, xanthan gum, or guar gum; carrageenan, dextran, gelatin, alginates, pectins, guar derivatives, xanthan derivatives or polysaccharides, such as starches, starch derivatives, cellulose ethers or cellulose esters. Preferred are carboxy-C₁-C₃-alkyl celluloses, or carboxy-C₁-C₃-alkyl hydroxy-C₁-C₃-alkyl celluloses, hydroxy-C₁₋₃-alkyl celluloses, such as hydroxyethyl celluloses; C₁-C₃-alkyl celluloses, such as methylcelluloses or ethylcelluloses; C₁-C₃-alkyl hydroxy-C₁₋₃-alkyl celluloses, such as hydroxyethyl methylcelluloses, hydroxypropyl methylcelluloses or ethyl hydroxyethyl celluloses. Other examples of useful polymers are homo- or copolymers of ethylene imine, an unsaturated acid, such as acrylic acid or a salt thereof, an unsaturated amide, such as acrylamide, a vinyl polymer, such as vinylalcohol, a vinyl ester, such as vinylacetate, vinylpyrrolidone, vinyloxazolidone, vinylmethyloxazolidone, ethylene sulfonic acid, vinylamine, vinylpyrridine or an alkylglycol. The film of the present invention can comprise one or more strengthening materials, such as nylon gauze, rayon mesh, Dacron or cellulose mesh. The amount of such other polymers or strengthening materials is preferably not more than 60 percent, more preferably not more than 40 percent, most preferably not more than 10 percent, based on the total weight of the film.

The film of the present invention preferably comprises a photoinitiator, such as benzoin, benzoin methyl ether, acetophenone, tetramethyldiaminobenzophenone (Michler's ketone) or the photoinitiators sold by Ciba-Geigy Specialty Chemicals, Switzerland, under the trademarks IRGACURE or DAROCUR. A preferred photoinitiator is benzophenone. If present, the weight of the photoinitiator in the film preferably is up 10 percent, more preferably from 0.4 to 5 percent, most preferably from 1 to 2 percent, based on the total weight of the alkylene oxide homo- or copolymer.

The film of the present invention can comprise further additives, such as one or more fillers or active ingredients, such as chemotherapeutic agents or medicaments. Useful therapeutic agents and other additives are for example those listed in column 7, lines 30 - 57 of U.S. Patent No. 3,419,006, the teaching of which is incorporated herein by reference. Their amount, if present, is generally not more than 20 percent, in most cases not more than 10 percent, based on the total weight of the film.

The film preferably has a thickness of from 20 micrometers to 5 millimeters, more preferably from 100 micrometers to 2.5 millimeters, most preferably from 200 to 500 micrometers. The film can be of any shape, for example in the shape of sheets or strips.

The film of the present invention can be produced in a process which comprises the steps of a) preparing a film comprising an alkylene oxide homo- or copolymer, b) shielding the film at one or both sides of the film at selected areas from irradiation such that a coherent pattern of non-shielded film material remains and c) subjecting the film to irradiation at one or both sides of the film.

Step a) of the process can be conducted in a known manner. For example, the film can be produced by compression-molding or extruding an alkylene oxide homo- or copolymer, optionally blended with one or more additives. Alternatively, an alkylene oxide homo- or copolymer, optionally blended with one or more additives, can be dissolved in water or an organic solvent, for example a chlorinated hydrocarbon, such as methylene chloride, or an alcohol, such as ethanol, or a mixture thereof. A film can then be casted from the solution by a known solvent casting method.

In step b) of the process of the present invention the film is shielded at selected areas from irradiation such that a coherent pattern of non-shielded film material remains. The film can be shielded at one or both sides in a known manner. For example, selected areas can be covered by patches of a material that does not allow a substantial transmission of the beams used in the radiation treatment, such as carton, glass, or a non-transparent plastic material.

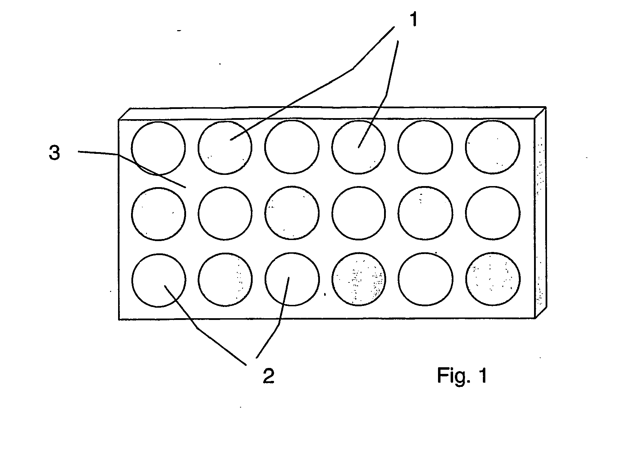

One preferred pattern of shielding the film to create the desired pattern for partial irradiation is illustrated in Figure 1. After cross-linking the film will comprise non-crosslinked areas 1 on the backside of the film and non-crosslinked areas 2 on the front side of the film. These non-crosslinked areas provide adhesive properties to the film. After cross-linking the film will also comprise a coherent pattern of cross-linked polymer 3, which provides the desired film integrity. If the film comprises non-crosslinked areas on both sides of the film, these areas are preferably arranged in a alternating manner such that each film area is subjected to irradiation from at least one side. An alternating pattern of non-crosslinked areas provides an optimum combination of film adhesiveness and film integrity.

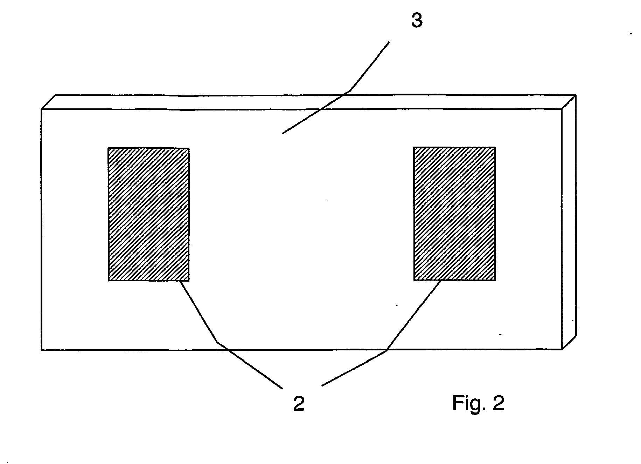

A significant advantage of the process of the present invention is that the areas of non-crosslinked and cross-linked polymer can be varied to a large degree and can be adjusted to the specific end-uses of the film. Depending on the intended end-use, it may be desirable to shield selected film areas from irradiation only at one side of the film. Such preferred embodiment of the present invention is illustrated in Fig. 2 wherein the film comprises non-crosslinked areas 2 on the front side of the film and a coherent pattern of cross-linked polymer 3.

In step c) of the process of the present invention the film is subjected to irradiation. Useful radiation treatments are, for example, gamma or electron beams, ionizing radiation including alpha particles, beta particles, X-rays, gamma rays and electrons from Van De Graff and other high voltage accelerators. A preferred treatment is irradiation with ultraviolet light. At least one, preferably both sides of the film are irradiated. It is within the knowledge of the skilled artisan to provide useful energy doses for irradiation of the film. For example, when using the commercially available Fusion System EPIQ 6000 equipment, an energy dose of 600 mJ/cm² to 19,000 mJ/cm², more preferably 1,200 mJ/cm² to 10,000 mJ/cm², and most preferably 3,000 to 6,000 mJ/cm² can be used to crosslinked the films. The energy for cross-linking is preferably chosen that the alkylene oxide homo- or copolymer in the cross-linked areas of the film has a water-solubility of less that 5 grams, preferably less than 2 grams, more preferably less than 1 gram in 100 grams of distilled water at 25 °C and 1 atmosphere.

The film of the present invention is useful as a stand-alone film or as a part of a composite article, for example as a wound dressing, for drug delivery, e.g., for dermatology or as controlled-release drug delivery system; as phase transfer catalysts, as semi-permeable membranes; or as an adhesive.

A composite article can for example contain the film of the present invention and a backing of a second polymeric material, such as polyvinylidene chloride, polyethylene, polypropylene, polyethylene terephthalate, polyamides, polyvinyl chloride, cellulose acetate and derivatives thereof, polydimethylbutadiene, polyurethanes, polyvinylalcohol, silicone rubber or polyacrylic acid. Due to the adhesive properties of the film of the present invention, generally no additional adhesive is necessary to fix the film of the present invention to a second polymeric material.

The present invention is illustrated by the following examples which should not be construed to limit the scope of the invention. Unless otherwise indicated, all parts and percentages are by weight.

### Examples 1 and 2 and Comparative Examples A - C

### Preparation and Cross-linking of the Film Samples

62.4 parts of a polyethylene oxide having a weight average molecular weight of about 2,000,000 and a viscosity of about 3,000 mPa.s, measured as a 2 weight percent aqueous solution, and 1.2 parts ofbenzophenone are dry blended in a low shear blender for 5 minutes. The polyethylene oxide is commercially available from Amerchol Corporation under the Trademark POLYOX N-60K Water Soluble Resin. A solution of 0.1 parts of Vitamin E in 9.1 parts of isopropyl alcohol is added to the powder mixture. After dry blending, the mixture is charged into a Brabender mixing bowl at 95°C. 18.2 parts of water and 9.1 parts of glycerin are added as plasticizers to the mixture in the Brabender mixing bowl. The molten mixture is then transferred to a hydraulic press and compression molded into a 10 mil (0.25 mm) film at 95°C and 2500psi (17.2 MPa). Film samples are collected and kept in polyethylene bags after they have been cooled in the press at room temperature.

Circular cartons of 1.2 mm are alternatingly stuck to both sides of the film samples to create the desired pattern for partial irradiation. The pattern is illustrated in Figure 1. Each film sample is placed on a conveyor belt and passed under a UV lamp in a Fusion System EPIQ6000 for ultraviolet curing. An energy level of 1050 mJ/cm² and a conveyor belt speed of 20 ft/minute (608 cm/min) are chosen for each irradiation pass. Two sets of films are irradiated. For the first set, each side of the film is passed under the UV lamp 5 times, such that the total curing energy is 5250 mJ/cm². This first set of films is identified hereafter as 5/5 film samples or Example 1. For the second set, each side of the film is passed under the UV lamp 3 times, such that the total curing energy is 3150 mJ/cm². This second set of films is identified hereafter as 3/3 film samples or Example 2.

### Adhesion Properties

The adhesion properties of the produced film samples are measured by using a Texture Analyzer from Texture Technologies Corporation. To perform the test, a 2 inch x 2 inch (5 cm x 5 cm) partially crosslinked film is adhered to a platform by double sided adhesive tape. The surface of the film is wetted twice with water. After thirty seconds hydration time an acrylic cylindrical probe of 1 inch (2.5 cm) diameter is pressed on the film sample with 500 grams force for 10 seconds. The film sample and the probe are aligned such that a non-crosslinked circle at the center of the film is aligned with the center of the probe. The bond strength measurement is made as the acrylic cylindrical probe is pulled away from the film after the 10 seconds contact time. Table I below summarizes the test results.

**Table 1**

| (Comparative) Example | A | 1 | 2 | B |
|---|---|---|---|---|
| Sample | 5/5 completely cross-linked* | 5/5 partially cross-linked | 3/3 partially cross-linked | 3/3 completely cross-linked* |
| | Adhesion (grams) | | | |
| 1 | 66.4 | 841.1 | 665.8 | 77.3 |
| 2 | 80.1 | 513.1 | 638.2 | 194.9 |
| 3 | 77.6 | 379.7 | 422.8 | 79.3 |
| 4 | 112.7 | 701.8 | 469.9 | 63.6 |
| 5 | 61.8 | 364.8 | 872.3 | 93.2 |
| 6 | 84.4 | 454.1 | 584.7 | 189.5 |
| 7 | 23.7 | 395.1 | 636.3 | 145.0 |
| 8 | 8.5 | 283.0 | 523.9 | 51.0 |
| Average | 64.4 | 491.6 | 601.7 | 111.7 |

| | | | | |
|---|---|---|---|---|
| * cross-linking by UV radiation without shielding any areas of the film | | | | |

The films of Examples 1 and 2 have good adhesion properties, but the films of Comparative Examples A and B have adhesion values which are too low for many end-uses which require film adhesiveness.

### Film solubility and Swell Ratio

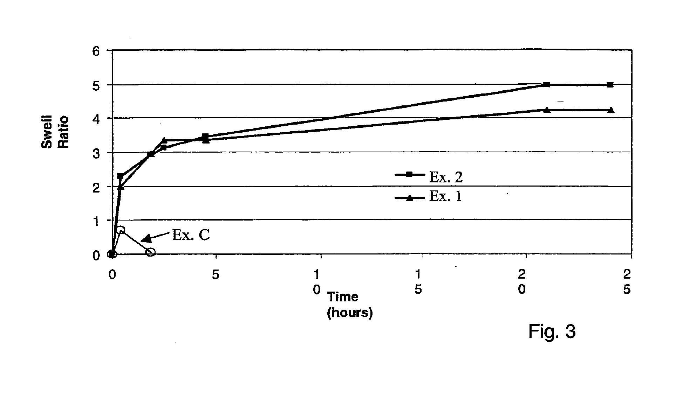

Figure 3 illustrates the water solubility of the partially crosslinked films of Examples 1 and 2, as compared with a non-crosslinked film of Comparative Example C which has not been subjected to irradiation. The data are generated by first measuring the area (length x width) of the dry film samples prior to water contact. Then each dry film sample is placed on a wire mesh screen and completely submerged in water during the test period. The area of the wet film is measured at the designated time intervals. The swell ratio of the film represents the area of a swollen film sample divided by the area of a dry film sample at the designated time intervals. Figure 3 illustrates that the non-crosslinked film of Comparative Example C dissolves quickly in water. It is not useful for end-uses where film integrity is required.

## Claims

1. A polymeric film comprising an alkylene oxide homo- or copolymer, wherein the film comprises i) a coherent pattern of cross-linked alkylene oxide homo- or copolymer and ii) areas of non-crosslinked alkylene oxide homo- or copolymer at one or both sides of the film.

2. The film of claim 1 wherein the alkylene oxide homo- or copolymer is a C₂₋₄ alkylene oxide homo- or copolymer.

3. The film of Claim 1 comprising at least 40 weight percent of an ethylene oxide homo- or copolymer, based on the total weight of the film.

4. The film of any one of Claims 1 to 3 additionally comprising a photoinitiator.

5. The film of Claim 4 wherein the photoinitiator is a benzophenone.

6. The film of Claim 4 or Claim 5 comprising from 0. 4 to 5 weight percent of a photoinitiator, based on the total weight of the alkylene oxide homo- or copolymer.

7. A composite article comprising the film of any one of Claims 1 to 6.

8. A process for preparing the film of any one of claims 1 to 6 comprising the steps of a) preparing a film comprising an alkylene oxide homo- or copolymer, b) shielding the film at one or both sides of the film at selected areas from irradiation such that a coherent pattern of non-shielded film material remains and c) subjecting the film to irradiation at one or both sides of the film.

9. The process of Claim 8 wherein the film is irradiated at both sides of the film.

10. Use of the film of any one of Claims 1 to 6 as an adhesive film or for preparing an adhesive film.

11. Use of the film of any one of Claims 1 to 6 for preparing a wound dressing film or a drug delivery film.

## Patentansprüche

1. Eine Polymerfolie, umfassend ein Alkylenoxid-Homo- oder Copolymer, wobei die Folie umfasst i) ein kohärentes Muster von vernetztem Alkylenoxid-Homo- oder Copolymer und ii) Bereiche von nicht vernetztem Alkylenoxid-Homo- oder Copolymer auf einer oder beiden Seiten der Folie.

2. Die Folie gemäss Anspruch 1, wobei das Alkylenoxid-Homo- oder Copolymer ein C₂₋₄-Alkylenoxid-Homo- oder Copolymer ist.

3. Die Folie gemäss Anspruch 1, umfassend zumindest 40 Gewichtsprozent eines Ethylenoxid-Homo- oder Copolymers basiert auf dem Gesamtgewicht der Folie.

4. Die Folie gemäss einem der Ansprüche 1 bis 3, zusätzlich umfassend einen Photoinitiatoren.

5. Die Folie gemäss Anspruch 4, wobei der Photoinitiator ein Benzophenon ist.

6. Die Folie gemäss Anspruch 4 oder 5, umfassend zwischen 0.4 und 5 Gewichtsprozent eines Photoinitiators basiert auf dem Gesamtgewicht des Alkylenoxid-Homo- oder Copolymers.

7. Eine Verbundware, umfassend die Folie gemäss irgendeinem der Ansprüche 1 bis 6.

8. Ein Verfahren zur Herstellung der Folie gemäss irgendeinem der Ansprüche 1 bis 6, umfassend die Schritte, dass a) eine Folie umfassend ein Alkylenoxid-Homo- oder Copolymer hergestellt wird, b) die Folie auf einer oder beiden Seiten der Folie in ausgewählten Bereichen gegenüber Bestrahlung abgeschirmt wird, so dass ein kohärentes Muster von unabgeschirmtem Folienmaterial übrigbleibt, und c) die Folie auf einer oder beiden Seiten der Folie einer Bestrahlung ausgesetzt wird.

9. Das Verfahren gemäss Anspruch 8, wobei die Folie auf beiden Seiten der Folie bestrahlt wird.

10. Verwendung der Folie gemäss irgendeinem der Ansprüche 1 bis 6 als eine Klebefolie oder für die Herstellung einer Klebefolie.

11. Verwendung der Folie gemäss irgendeinem der Ansprüche 1 bis 6 für die Herstellung einer Wundverband-Folie oder einer Folie zur Medikamentenabgabe.

## Revendications

1. Film en polymère, comprenant un homopolymère ou copolymère d'oxyde d'alkylène, lequel film comporte :
i) un motif d'un seul tenant, en homopolymère ou copolymère d'oxyde d'alkylène réticulé,
ii) et, sur l'une de ses faces ou sur les deux, des zones en homopolymère ou copolymère d'oxyde d'alkylène non-réticulé.

2. Film conforme à la revendication 1, dans lequel l'homopolymère ou copolymère d'oxyde d'alkylène est un homopolymère ou copolymère d'oxyde d'alkylène en C₂₋₄.

3. Film conforme à la revendication 1, comprenant un homopolymère ou copolymère d'oxyde d'éthylène, en une quantité représentant au moins 40 % du poids total du film.

4. Film conforme à l'une des revendications 1 à 3, comprenant en outre un photoamorceur.

5. Film conforme à la revendication 4, dans lequel le photoamorceur est une benzophénone.

6. Film conforme à la revendication 4 ou 5, qui comprend un photoamorceur en une quantité représentant de 0,4 à 5 % du poids total de l'homopolymère ou copolymère d'oxyde d'alkylène.

7. Article composite comprenant un film conforme à l'une des revendications 1 à 6.

8. Procédé de préparation d'un film conforme à l'une des revendications 1 à 6, lequel procédé comporte les étapes suivantes :
a) préparer un film comprenant un homopolymère ou copolymère d'oxyde d'alkylène ;
b) protéger le film contre une irradiation, à l'aide d'un écran, en des zones sélectionnées sur l'une de ses faces ou sur les deux, de manière à laisser sans protection un motif d'un seul tenant dans le film ;
c) et irradier le film, sur l'une de ses faces ou sur les deux.

9. Procédé conforme à la revendication 8, dans lequel le film est irradié sur ses deux faces.

10. Emploi d'un film conforme à l'une des revendications 1 à 6, en tant que film adhésif ou en vue de la fabrication d'un film adhésif.

11. Emploi d'un film conforme l'une des revendications 1 à 6, en vue de la fabrication d'un film à pansement ou d'un film servant à administrer un médicament.
